(19) Europäisches Patentamt
European Patent Office
Office européen des brevets

(11) **EP 3 791 136 B1**

(12) **EUROPÄISCHE PATENTSCHRIFT**

(45) Veröffentlichungstag und Bekanntmachung des Hinweises auf die Patenterteilung:
**22.02.2023** **Patentblatt 2023/08**

(21) Anmeldenummer: **19724128.4**

(22) Anmeldetag: **06.05.2019**

(51) Internationale Patentklassifikation (IPC):
*G01D 5/14* *(2006.01)*   *A61F 5/01* *(2006.01)*

(52) Gemeinsame Patentklassifikation (CPC):
**G01D 5/145**

(86) Internationale Anmeldenummer:
**PCT/EP2019/061577**

(87) Internationale Veröffentlichungsnummer:
**WO 2019/215096 (14.11.2019 Gazette 2019/46)**

(54) **ORTHOPÄDISCHES HILFSMITTEL**

ORTHOPAEDIC AID

AIDE ORTHOPÉDIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.05.2018   DE 102018111234**

(43) Veröffentlichungstag der Anmeldung:
**17.03.2021   Patentblatt 2021/11**

(73) Patentinhaber: **Otto Bock Healthcare Products GmbH**
**1110 Wien (AT)**

(72) Erfinder:
• **EDER, Marcus**
**1090 Wien (AT)**
• **WAGNER, Wolfang**
**2485 Wimpassing (AT)**
• **INSCHLAG, Josef**
**8251 Bruck an der Lafnitz (AT)**
• **WASHÜTTL, Martin**
**2004 Niederhollabrunn (AT)**
• **WEBER, Martin**
**2301 Groß-Enzersdorf (AT)**
• **SEDLAK, Richard**
**1230 Wien (AT)**
• **AUBERGER, Roland**
**1140 Wien (AT)**

(74) Vertreter: **Gramm, Lins & Partner**
**Patent- und Rechtsanwälte PartGmbB**
**Frankfurter Straße 3 C**
**38122 Braunschweig (DE)**

(56) Entgegenhaltungen:
EP-A2- 2 792 306    DE-A1-102015 117 080
DE-T2- 69 331 195    GB-A- 2 367 753
US-A1- 2016 238 672    US-B2- 7 195 645

**Beschreibung**

Die Erfindung betrifft ein orthopädisches Hilfsmittel gemäß dem Oberbegriff von Anspruch 1. Gemäß einem zweiten Aspekt betrifft die Erfindung ein Verfahren zum Bestimmen einer Stellung eines derartigen orthopädischen Hilfsmittels.

Orthopädische Hilfsmittel sind beispielsweise Orthesen oder Prothesen, insbesondere Exo-Prothesen. Derartige orthopädische Hilfsmittel besitzen häufig Aktoren, mittels derer die Eigenschaften des orthopädischen Hilfsmittels in Abhängigkeit von einer Position des Bewegungselements relativ zum Bezugselement beeinflusst werden. Es ist daher notwendig, die Stellung des orthopädischen Hilfemittels, also die Position des Bewegungselements relativ zum Bezugselement, mit möglichst hoher Genauigkeit zu kennen.

Orthopädische Hilfsmittel verfügen aus Gewichtsgründen in der Regel nicht über große Energiespeicher, sodass die Bestimmung der Stellung des orthopädischen Hilfsmittels zudem mit möglichst geringem Energieaufwand erfolgen sollte.

Aus der US 2014/0025182 A1 ist eine Prothese bekannt, die einen Motor aufweist. Die Position des Motors wird mittels eines Hall-Sensors bestimmt, der ein Signal empfängt, wenn ein Magnetelement an ihm vorbeistreicht. Das Magnetelement ist auf dem Rotor des Motors positioniert. Nachteilig an einem derartigen orthopädischen Hilfsmittel ist, dass eine Geschwindigkeit und eine Beschleunigung des Bewegungselements relativ zum Bezugselement nur vergleichsweise ungenau bestimmt werden können.

Aus der US 2013/0245785 A1 ist eine Prothese bekannt, die eine Vakuumpumpe aufweist. Die Vakuumpumpe kann mit einem Schalter bedient werden, der einen Hall-Sensor aufweist. Wird an diesem Hall-Sensor ein magnetisches Element vorbeigeführt, so schaltet der Schalter. Ein derartiges System ist lediglich zu der binären Aussage in der Lage, ob der Schalter geschaltet ist oder nicht.

Aus der US 2016/0302946 A1 ist eine Prothese bekannt, die einen Lastsensor aufweist. Dieser Lastsensor umfasst einen Hall-Sensor und einen Magneten, der sich dann relativ zu dem Hall-Sensor bewegt, wenn eine Last an der Prothese anliegt. Mit einem derartigen System ist es zwar möglich, die Position der beiden Komponenten der Prothese relativ zueinander vergleichsweise gut zu bestimmen, dynamische Größen wie beispielsweise die Geschwindigkeit oder die Beschleunigung können jedoch nicht mit hinreichend hoher Genauigkeit bestimmt werden.

Aus der US 2018/0116826 A1 ist eine Prothese bekannt, bei der ebenfalls ein Hall-Sensor eingesetzt wird, mittels dem eine Position eines Rotors eines Motors relativ zum Stator bestimmt werden kann. Auch mit einem solchen System sind zwar möglicherweise hinreichend genau Positionsdaten ermittelbar, nicht aber hinreichend genaue Geschwindigkeits- und/oder Beschleunigungsdaten.

Aus der EP 2 696 814 B1 ist eine Prothesenvorrichtung bekannt, die eine Vielzahl an Antrieben aufweist. Es wird gelehrt, dass binäre und nicht-binäre Sensoren verwendet werden können, beispielsweise Beschleunigungsmesser oder Gyroskope. Derartige Sensoren sind für das Ermitteln der Relativposition und der Relativgeschwindigkeit zwischen Bewegungselement und Bezugselement nur wenig geeignet.

Aus der US 7 195 645 B2 ist eine Vorrichtung bekannt, um bei einer Endo-Knieprothese die Größe des Gelenkspalts zu bestimmen. Dazu ist in einem der Gelenkteile ein Magnet eingelassen. Im gegenüberliegenden Gelenkteil befindet sich ein Hall-Sensor, der das resultierende Magnetfeld misst. Bei diesem Hall-Sensor handelt es sich beispielsweise um einen linearen Hall-Sensor, dessen Messspannung linear vom gemessenen Magnetfeld abhängt. Mit einem derartigen Sensor ist die Stellung der Bezugselemente relativ zueinander, wenn sie sich entlang der Trajektorie bewegen, nicht ermittelbar.

Aus der US 2016/0238672 A ist ein Verfahren zum Bestimmen einer Relativposition einer Magnetfeldquelle bekannt. Die Messergebnisse, die mit einem Magnetfeldsensor aufgenommen werden, werden um den Temperatureinfluss korrigiert.

Aus der GB 2 367 753 A ist eine Knieprothese bekannt, die einen Zylinder aufweist, der zum Dämpfen der Bewegung dient. Ein Bestimmen einer Position eines ersten Bezugselements zu einem zweiten Bezugselement mittels Magneten und Roll-Sensoren erfolgt nicht.

Der Erfindung liegt die Aufgabe zugrunde, ein orthopädisches Hilfsmittel vorzuschlagen, bei dem die Stellung der Bezugselemente zueinander bei Bewegung entlang der Trajektorie genau bestimmbar ist.

Die Erfindung löst das Problem durch ein orthopädisches Hilfsmittels mit den Merkmalen von Anspruch 1.

Die Erfindung löst das Problem durch ein Verfahren zum Bestimmen der Stellung eines orthopädischen Hilfsmittels mit den oben genannten Eigenschaften, mit den Schritten (i) Erfassen einer ersten Hall-Spannung eines ersten Hall-Sensors und einer zweiten Hall-Spannung eines zum ersten Hall-Sensors benachbarten zweiten Hall-Sensors und (ii) Bestimmen der Position des Bewegungselements relativ zum Bezugselement aus einer Hallspannungs-Differenz, die gebildet ist aus erster Hall-Spannung und zweiter Hall-Spannung, sowie aus der zweiten Hall-Spannung. Insbesondere wird die Position des Bewegungselements aus der Hallspannungs-Differenz und einem Abstand zweier benachbarter Hall-Sensoren sowie einer dieser Hall-Spannungen berechnet. Das Bestimmen ist insbesondere ein Berechnen.

Vorteilhaft an einem derartigen orthopädischen Hilfsmittel ist, dass seine Stellung, das heißt die Position des Bewegungselements relativ zum Bezugselement, sowohl mit hoher Genauigkeit als auch mit vergleichsweise geringem Energieaufwand ermittelt werden kann. Aufgrund der hohen Messgenauigkeit ist es zudem möglich, durch automatisches Ableiten die Geschwindigkeit zu bestimmen, mit der sich das Bewegungselement relativ zum Bezugselement bewegt. Beim Ableiten vergrößert sich die Messunsicherheit deutlich, weshalb es notwendig ist, die Stellung des orthopädischen Hilfsmittels möglichst genau zu bestimmen. Das ist mit dem erfindungsgemäßen System möglich.

Vorteilhaft ist es zudem, dass die Bestimmung der Stellung robust möglich ist. Hall-Sensoren besitzen keine beweglichen Teile, sodass ein mechanischer Verschleiß klein ist. Es handelt sich bei Hall-Sensoren zudem um Standard-Bauteile, die mit einer hohen Genauigkeit in großen Mengen hergestellt werden. Das orthopädische Hilfsmittel kann daher verhältnismäßig günstig hergestellt werden.

Im Rahmen der vorliegenden Beschreibung wird unter einem orthopädischen Hilfsmittel insbesondere eine Vorrichtung verstanden, die dazu ausgebildet ist, mit einem menschlichem oder tierischem Körper verbunden zu werden, um die Funktion eines erkrankten oder nicht vorhandenen Gelenks oder der das Gelenk umgebenden Muskulatur zu ersetzen oder zu unterstützen.

Unter dem orthopädischen Hilfsmittel wird insbesondere eine Orthese oder eine Prothese, insbesondere eine Exo-Prothese, verstanden. Beispielsweise ist das orthopädische Hilfsmittel eine Knie-Exoprothese.

Unter dem Bezugselement wird insbesondere ein Bauteil des Hilfsmittels verstanden, zu dem das Bewegungselement eine Relativbewegung ausführt. Es sei drauf hingewiesen, dass es sich um eine relative Bewegung von Bewegungselement zu Bezugselement handelt. Aus diesem Grund könnte das Bewegungselement auch als Bezugselement und das Bezugselement auch als Bewegungselement aufgefasst werden. Die Benennung soll lediglich die Erläuterung der Erfindung erleichtern. Da es lediglich auf die Relativbewegungen zwischen dem Bezugselement und dem Bewegungselement ankommt, wird dasjenige Element, an dem die Hall-Sensoren befestigt sind, als Bezugselement betrachtet. Statt des Begriffs Bezugselement könnte auch der Begriff erstes Element verwendet werden und statt des Begriffs Bewegungselement könnte auch der Begriff zweites Element verwendet werden.

Unter dem Merkmal, dass das Bewegungselement bewegbar am Bezugselement befestigt ist, wird insbesondere verstanden, dass beide aneinander definiert geführt befestigt sind.

Unter dem Positionssensor wird insbesondere jede Vorrichtung verstanden, mittels der die Position des Bewegungselements relativ zum Bezugselement automatisch feststellbar ist. Vorzugsweise gibt der Positionssensor ein elektrisches Signal ab, das die Position des Bewegungselements relativ zum Bezugselement kodiert. Es ist dabei möglich, nicht aber notwendig, dass dieses elektrische Signal die Position in absoluten Einheiten, insbesondere SI-Einheiten, angibt. Insbesondere ist es auch möglich, dass die Position in einem für das das orthopädische Hilfsmittel spezifischen Koordinatensystem und/oder Einheitensystem angegeben ist.

Unter dem Merkmal, dass die Hall-Sensoren am Bewegungselement zum Bewegen entlang einer Trajektorie zum Bewegen des Bewegungselement relativ zum Bezugselement angeordnet sind, wird insbesondere verstanden, dass eine Bewegung des Bewegungselements zum ruhend gedachten Bezugselement dazu führt, dass sich die Hall-Sensoren entlang einer Kurve, nämlich der Trajektorie, bewegen. Insbesondere bewegen sich alle Hall-Sensoren entlang der gleichen Trajektorie. Bei der Trajektorie kann es sich beispielsweise um einen Kreis oder eine Gerade handeln, das ist aber nicht notwendig. Insbesondere kann die Trajektorie beispielsweise auch eine Ellipse sein oder eine andere Gestalt haben.

Unter dem Merkmal, dass die Hall-Sensoren so angeordnet sind, dass eine Bewegung der Hall-Sensoren entlang der Trajektorie für zumindest einen Hall-Sensor eine lineare Änderung seiner Hall-Spannung bewirkt, wird insbesondere verstanden, dass stets ein Hall-Sensor existiert, für den diese Forderung gilt. Insbesondere gilt diese Forderung in der Regel nicht für alle Hall-Sensoren gleichzeitig, sondern lediglich für jeweils zwei Hall-Sensoren unabhängig von der Stellung des Bewegungselements relativ zum Bezugselement. Unter einer linearen Änderung der Hall-Spannung wird eine lineare Änderung im technischen Sinne verstanden. In anderen Worten ist es möglich, dass die Hall-Spannung nicht im mathematischen Sinne linear von der Änderung der Position des Hall-Sensors abhängt, solange diese Abweichung hinreichend klein ist.

Selbstverständlich kann in der Regel jede Kurve in erster Näherung als linear betrachtet werden, das ist aber mit dem vorliegenden Merkmal nicht gemeint. Vielmehr sind die Hall-Sensoren so angeordnet, dass ein Messfehler, der durch die Annahme der Linearität der Änderung der Hall-Spannung in Abhängigkeit von einer Positionsänderung kleiner als ein vorgegebener Wert ist, der bevorzugt kleiner als 2 % ist.

Besonders günstig ist es, wenn die Hall-Sensoren temperaturkompensiert sind.

Unter dem Merkmal, dass der erste Magnetschenkel sich in die Magnetschenkel-Richtung erstreckt, wird insbesondere verstanden, dass der erste Magnetschenkel benachbart zu seinem freien Ende sich in diese Richtung erstreckt. Wenn der Magnetschenkel prismatisch ist, insbesondere quaderförmig, so entspricht die Magnetschenkel-Richtung der Extrusionsrichtung des Prismas.

Unter dem Merkmal, dass die freien Enden, angebracht am Bewegungselement, sich entlang der Trajektorie angeordnet sind oder bewegen, wird insbesondere verstanden, dass die beiden Enden den gleichen Abstand von der Trajektorie haben. Unter dem Abstand wird - wie allgemein üblich - die Länge der kürzesten Strecke verstanden, die zwei Objekte miteinander verbindet. Unter dem gleichen Abstand wird der im technischen Sinne gleiche Abstand verstanden. Es ist daher möglich, nicht aber notwendig, dass der Abstand im mathematischen Sinne gleich ist, es sind aber auch relative Abweichungen von beispielsweise maximal 10% möglich

Gemäß einer bevorzugten Ausführungsform besitzt der Permanentmagnet (a) ein Magnetformflussteil, das ein Weichmagnet-Element, das aus einem weichmagnetischen Werkstoff besteht, (b) einen ersten Teil-Permanentmagneten, der den ersten Magnetschenkel bildet und mit seinem dem ersten freien Ende gegenüberliegenden ersten Kontakt-Ende am Weichmagnet-Element anliegt, (c) einen zweiten Teil-Permanentmagneten, der den zweiten Magnetschenkel bildet und mit seinem dem zweiten freien Ende gegenüberliegenden zweiten Kontaktende am Weichmagnet-Element anliegt und (d) einen dritten Teil-Permanentmagneten, der zwischen dem ersten Teil-Permanentmagneten und dem zweiten Teil-Permanentmagneten angeordnet ist, quer zum ersten Magnetschenkel und zum zweiten Magnetschenkel verläuft und eine magnetische Drittpermanentmagnet-Orientierung hat, die quer zu einer Erstpermanentmagnet-Orientierung des ersten Teil-Permanentmagneten verläuft und quer zu einer Zweitpermanentmagnet-Orientierung des zweiten Teil-Permanentmagneten verläuft.

Es hat sich herausgestellt, dass ein so aufgebauter Permanentmagnet ein besonders homogenes Feld am Ort der Hall-Sensoren erzeugt.

Vorzugsweise weist das Magnetflussformteil ein Nichtferromagnetteil auf, das im Magnetflusslinienverlauf zwischen dem ersten Teil-Permanentmagneten und dritten Teil-Permanentmagneten angeordnet ist und/oder das im Magnetflusslinienverlauf zwischen dem zweiten Teil-Permanentmagneten und dem dritten Teil-Permanentmagneten angeordnet ist. Unter dem Merkmal, dass das Nichtferromagnetteil im Magnetflusslinienverlauf zwischen dem ersten und dem dritten Teil-Permanentmagneten angeordnet ist, wird insbesondere verstanden, dass die Magnetflusslinien vom ersten Teil-Permanentmagneten durch das Nichtferromagnetteil zum dritten Teil-Permanentmagneten verlaufen. Das Nichtferromagnetteil besteht aus, insbesondere diamagnetischem oder paramagnetischem, Material, das nicht ferromagnetisch ist, beispielsweise einem Metall, insbesondere aus Kupfer oder aus Kunststoff. Die Dicke des Nichtferromagnetteils ist so gewählt, dass die Hall-Sensoren über einen möglichst weiten Bereich der Bewegung entlang der Trajektorie für zumindest einen Hall-Sensor eine annähernd lineare Änderung der Hall-Spannung zeigen. Die ideale Dicke wird in Vorversuchen ermittelt.

Vorzugsweise hat der Permanentmagnet eine Permanentmagnet-Länge entlang der Trajektorie, die zumindest doppelt, insbesondere dreifach so groß ist wie ein Hall-Sensoren-Abstand zweier benachbarter Hall-Sensoren. Auf diese Weise ergibt sich am Ort des Hall-Sensors ein hinreichend homogenes Magnetfeld, sodass eine hohe Messgenauigkeit erreichbar ist.

Unter dem Abstand zweier Hall-Sensoren wird insbesondere diejenige Strecke verstanden, um die die Hall-Sensoren bewegt werden müssen, bis der benachbarte Hall-Sensor an der gleichen Stelle angeordnet ist wie der vorherige Hall-Sensor.

Vorzugsweise beträgt ein Abstand der Trajektorie vom Permanentmagneten höchstens die Hälfte der Permanentmagnet-Länge. Das führt zu einem hinreichend homogenen Magnetfeld bei den Hall-Sensoren. Günstig ist es zudem, wenn der Abstand zumindest ein Zehntel der Permanentmagnet-Länge beträgt.

Vorzugsweise besitzt das orthopädischen Hilfsmittel eine elektrische Auswerteeinheit, die ausgebildet ist zum automatischen Durchführen eines Verfahrens mit den Schritten (i) Erfassen einer ersten Hall-Spannung eines ersten Hall-Sensors und einer zweiten Hall-Spannung eines zum ersten Hall-Sensors benachbarten zweiten Hall-Sensors und (ii) Bestimmen einer Position des Bewegungselements relativ zum Bezugselement aus einer Hallspannungs-Differenz aus erster Hall-Spannung und zweiter Hall-Spannung.

Aus den Hall-Spannungen, die an den Hall-Sensoren anliegen, könnte bereits die Position der Hall-Sensoren relativ zum Permanentmagneten und damit die Position des Bewegungselements relativ zum Bezugselement bestimmt werden. Es hat sich jedoch herausgestellt, dass die zusätzliche Betrachtung der Hallspannungs-Differenz die Bestimmung der Position des Bewegungselements relativ zum Bezugselement mit einer höheren Genauigkeit gestattet.

Vorzugsweise ist die elektrische Auswerteeinheit ausgebildet zum automatischen Durchführen eines Verfahrens mit den Schritten (i) Erfassung der Hall-Spannung von zumindest drei Hall-Sensoren, (ii) Erfassen derjenigen Hall-Sensoren, bei denen die Hall-Spannungen die betragsmäßig kleinsten Werte annehmen und (iii) Bestimmen der Position des Bewegungselements relativ zum Bezugselement aus den Positionen dieser Hall-Sensoren und einer Hallspannungs-Differenz dieser Hall-Spannungen. Bei korrekt positionierten Hall-Sensoren verschwindet die Hall-Spannung, wenn das anliegende Magnetfeld keine Normalkomponente auf die Sensorebene hat. Das ist vorzugsweise dann der Fall, wenn sich der Hall-Sensor genau zwischen den beiden Magnetschenkeln befindet. Eine Auslenkung aus dieser Position führt zu einer in guter Näherung linearen Änderung der Hall-Spannung. Unter einer in guter Näherung linearen Änderung wird verstanden, dass die Abweichung zum linearen Verhalten höchstens 2% beträgt.

Zur Berechnung der Hallspannungs-Differenz zum benachbarten Hall-Sensor wird vorzugsweise diejenige Hall-Spannung verwendet, die betragsmäßig am kleinsten ist. Diese Spannung gehört zu demjenigen Hall-Sensor, dessen Abstand zu der oben genannten Position zwischen den beiden Magnetschenkeln kleiner ist als des anderen benachbarten Hall-Sensors. Auf diese Weise ist sichergestellt, dass stets die beiden Hall-Sensoren zur Bestimmung der Stellung des orthopädischen Hilfsmittels verwendet werden, die den kleinsten Abstand von der Position zwischen den beiden Magnetschenkeln haben. So wird eine besonders hohe Genauigkeit bei der Positionsmessung erreicht.

Günstig ist es, wenn das Bezugselement ein Zylinder ist und das Bewegungselement ein Kolben ist, der im Zylinder läuft, wobei der Positionssensor ein Kolben-Positionssensor zum Messen einer Position des Kolbens im Zylinder ist und wobei der Permanentmagnet am Kolben angeordnet ist und der Kolben-Positionssensor am Zylinder angeordnet ist. Auf diese Weise kann die Position des Kolbens im Zylinder mit hoher Genauigkeit gemessen werden.

Alternativ oder zusätzlich ist das Bezugselement ein erster Schenkel, das Bewegungselement ein zweiter Schenkel, wobei er erste Schenkel und der zweite Schenkel mittels eines Gelenks, insbesondere eines Drehgelenks, miteinander verbunden sind und der Positionssensor ein Schenkel-Winkelsensor zur Messung einer Winkelstellung des ersten Schenkels relativ zum zweiten Schenkel ist.

Gemäß einer bevorzugten Ausführungsform ist die Auswerteeinheit ausgebildet zum Abschalten von solchen Hall-Sensoren, deren Messergebnisse nicht an der Berechnung der Position des Bewegungselements beteiligt sind. Auf diese Weise wird der Energieverbrauch klein gehalten.

Erfindungsgemäß ist zudem ein Verfahren zum Bestimmen einer Stellung eines orthopädischen Hilfsmittels mit einem Bezugselement, (b) einem Bewegungselement, (c) einem Positionssensor zum Bestimmen einer Position des Bewegungselements relativ zum Bezugselement, der zumindest einen Permanentmagneten und zumindest drei Hall-Sensoren aufweist, (d) wobei die Hall-Sensoren am Bezugselement angeordnet sind und das Bewegungselement zum Bewegen entlang einer Trajektorie relativ zum Bezugselement angeordnet ist, mit den Schritten: (i) Erfassen einer ersten Hall-Spannung eines ersten Hall-Sensors und einer zweiten Hall-Spannung eines zum ersten Hall-Sensors benachbarten zweiten Hall-Sensors und (ii) Bestimmen der Position des Bewegungselements relativ zum Bezugselement aus einer Hallspannungs-Differenz aus erster Hall-Spannung und zweiter Hall-Spannung und der zweiten Hall-Spannung.

Vorzugsweise umfasst das Verfahren die Schritte: (i) Erfassen der Hall-Spannungen von zumindest drei Hall-Sensoren, (ii) Erfassen derjenigen, insbesondere zwei, Hall-Sensoren, bei denen die Hall-Spannungen die betragsmäßig kleinsten Werte annehmen, und (iii) Bestimmen der Position des Permanentmagneten aus den Positionen dieser Hall-Sensoren und einer Hallspannungs-Differenz dieser Hall-Spannungen. Sind zwei Hall-Spannungen gleich, wir einer der beiden Hall-Sensoren ausgewählt, beispielsweise der mit einer kleineren Kennzahl, wobei in diesem Fall alle Hall-Sensoren eine Kennzahl haben und nach Größe der Kennzahl sortiert angeordnet sind.

Vorzugsweise umfasst das Verfahren die Schritte: (i) für jeden Hall-Sensor Erfassen einer Offset-Spannung der Hall-Spannung, die verursacht ist vom Vorhandensein eines Winkels zwischen einer Ist-Lage des Hall-Sensors und einer Soll-Lage und (ii) Korrigieren der Hall-Spannung um die Offset-Spannung. Die Soll-Lage ist insbesondere diejenige, bei der keine Hall-Spannung am Hall-Sensor anliegt, wenn sich der Hall-Sensor genau zwischen den beiden Magnetschenkeln befindet. Ist der Hall-Sensor zu dieser Soll-Lage verkippt montiert, so ergibt sich auch in dieser Position eine Normalkomponente des Magnetfelds. Diese Normalkomponente ist die gleiche, die bei einer Bewegung entlang der Trajektorie entstehen würde. Es ist daher vorteilhaft, diese Offset-Spannung von der gemessenen Hall-Spannung abzuziehen. Zum Durchführen dieser Korrektur besitzt die elektrische Auswerteeinheit vorzugsweise einen digitalen Speicher, in dem für jeden Hall-Sensor die Offset gespeichert ist und die Auswerteeinheit ist ausgebildet zum automatischen Abziehen der Offset-Spannung von der gemessenen Hall-Spannung.

Diese Offset-Spannung wird beispielsweise dadurch gemessen, dass die Spannung gemessen wird, wenn am Hall-Sensor kein Magnetfeld anliegt. Die beim späteren Einsatz gemessenen Hall-Spannungen $U_{Hall,N}$ werden bei der Auswertung um den Wert der Offset-Spannung korrigiert. $U'_{Hall,N}$ entspricht dem so korrigierten Wert der Hall-Spannung.

Gemäß einer bevorzugten Ausführungsform umfasst das Verfahren die Schritte (i) für jeden Hall-Sensor Erfassen einer Sensitivität, die die Abhängigkeit der Hall-Spannung vom Magnetfeld beschreibt, und (ii) Korrigieren der Position um den Einfluss der Sensitivität. Die Sensitivität wird gemessen durch Anlegen eines bekannten Magnetfelds an den Hall-Sensor und Messen der entstehenden Hall-Spannung. Die so ermittelten Sensitivitäten werden für alle Hall-Sensoren digital in der Auswerteeinheit gespeichert.

Beispielsweise werden die Hall-Sensoren in einer Prüfmaschine kalibriert. Dabei wird ein Digitalinkrementalgeber an einen Motor angeflanscht. Der Motor bewegt einen Referenzmagneten über die Hall-Sensoren im Kreis. Der Inkrementalgeber liefert jeweils den genauen Momentanwinkel, die Sensoren liefern die Hall-Spannungen.

Dabei werden die Kurven für alle Hallsensoren 42.i aufgezeichnet. Die Sensitivität ist die Steigung der Kurve, die die Hall-Spannung gegen das Magnetfeld aufträgt.

Im Folgenden wird die Erfindung anhand der beigefügten Zeichnungen näher erläutert. Dabei zeigt

Figur 1     eine Seitenansicht eines erfindungsgemäßen orthopädischen Hilfsmittels in Form einer Knie-Exoprothese,

Figur 2a     einen Ausschnitt aus dem orthopädischen Hilfsmittel gemäß Figur 1, in dem das Bezugselement, das Bewegungselement und der Positionssensor zu sehen sind,

Figur 2b    das orthopädischen Hilfsmittel gemäß Figur 2a, bei dem das Bewegungselement teilweise entfernt ist,

Figur 3a    eine schematische Seitenansicht eines Permanentmagneten des orthopädischen Hilfsmittels gemäß der Figuren 1 und 2,

Figur 3b    eine perspektivische Ansicht des Permanentmagneten gemäß Figur 3a,

Figur 3c    den Magnetlinienverlauf des Permanentmagneten gemäß der Figuren 3a und 3b,

Figur 4a    eine Ansicht des Magnetlinienverlaufs des Permanentmagneten im Verhältnis zu einem Hall-Sensor,

Figur 4b    die Abhängigkeit der Hall-Spannung von einer Position eines Hall-Sensors relativ zum Permanentmagneten,

Figur 5a    die Hallspannungsverläufe von drei Hall-Sensoren zur Erläuterung der Positionsbestimmung der Hall-Sensoren relativ zum Permanentmagneten,

Figur 5b    den Verlauf der Hall-Spannungen für mehrere Hall-Sensoren in Abhängigkeit von der Position des Bewegungselements relativ zum Bezugselement zum Erläutern der Positionsberechnung und

Figur 6    einen Zylinder eines erfindungsgemäßen orthopädischen Hilfsmittels, bei dem der Positionssensor zum Bestimmen der Position des Zylinders im Kolben ausgebildet ist.

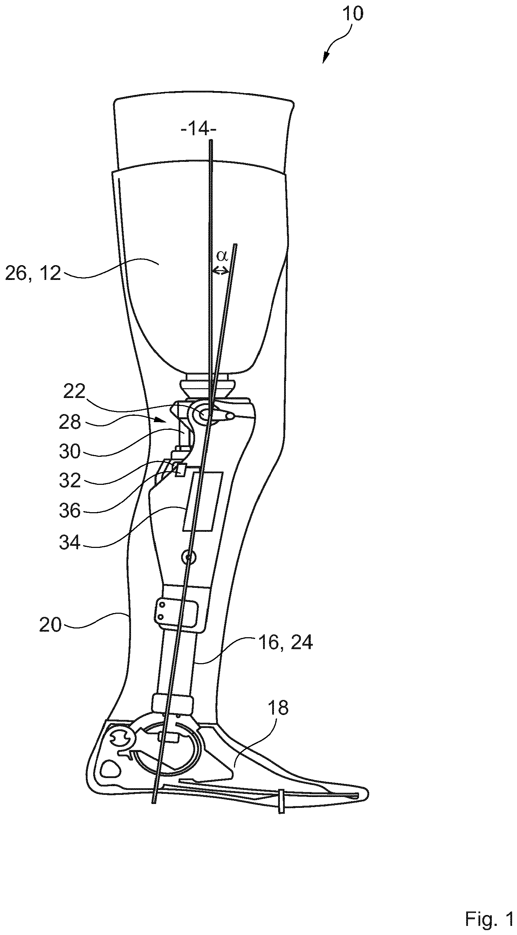

Figur 1 zeigt ein erfindungsgemäßes orthopädisches Hilfsmittel 10 in Form einer Knie-Exoprothese, die einen Schaft 12 zur Aufnahme eines menschlichen Oberschenkelstumpfs 14 und einen künstlichen Unterschenkel 16 aufweist. Der Unterschenkel 16 ist mit einem künstlichen Fuß 18 verbunden. Es ist möglich und bevorzugt, dass das orthopädische Hilfsmittel eine Kosmetik 20 besitzt, die der Knie-Exoprothese eine natürliche Anmutung verleiht.

Das Hilfsmittel 10 besitzt ein Drehgelenk 22, um das der Unterschenkel 16 relativ zum Schaft 12 um einen Schwenkwinkel $\alpha$ schwenken kann. Im vorliegenden Fall stellt der Schaft 12 ein Bezugselement 26 dar, relativ zu dem sich ein Bewegungselement 24 in Form des Unterschenkels bewegen kann.

Im vorliegenden Fall weist das Hilfsmittel 10 einen Dämpfer 28 auf, der einen Kolben 30 besitzt, der in einem Zylinder 32 läuft. Je nach Stellung des Bewegungselements 24 relativ zum Bezugselement 26 ändert sich die Stellung des Kolbens 30 im Zylinder 32.

Am orthopädischen Hilfsmittel 10 gemäß Figur 1 wird deutlich, dass lediglich eine Relativbewegung zwischen dem Bewegungselement 24 und dem Bezugselement 26 relevant ist. Zudem bewegt sich das Bezugselement 26 bei Benutzung des Hilfsmittels 10 auch.

Das Hilfsmittel 10 umfasst eine schematisch eingezeichnete Auswerteeinheit 34, die, möglich, nicht aber notwendig ist, mit einem schematisch eingezeichneten Aktor 36 verbunden ist. Mittels des Aktors 36 sind die Dämpfeigenschaften des Dämpfers 28 veränderbar. Insbesondere kann der Dämpfer vorzugsweise gesperrt werden, sodass der Kolben 30 sich nicht mehr im Zylinder 32 bewegen kann. Alternativ oder zusätzlich kann mittels des Aktors die Kraft verändert werden, die am Kolben 30 anliegen muss, um diesen mit einer vorgegebenen Geschwindigkeit relativ zum Zylinder 32 zu bewegen.

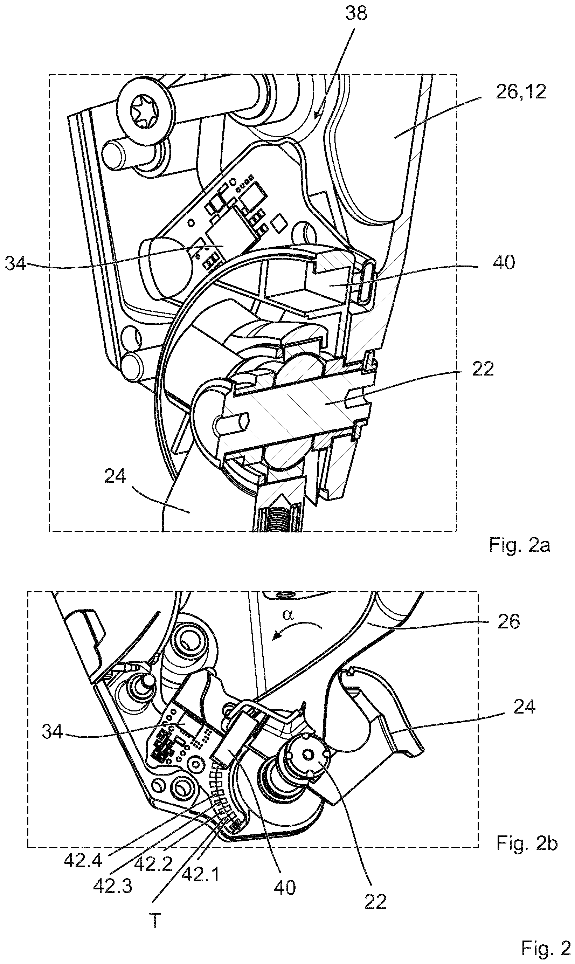

Figur 2a zeigt eine geschnittene Teil-Ansicht des Hilfsmittels 10. Es ist zu erkennen, dass das Hilfsmittel 10 einen Positionssensor 38 aufweist. Der Positionssensor 38 umfasst Hall-Sensoren 42.i (i= 1, 2, ..., 18) und die Auswerteeinrichtung 34, die am Bezugselement 26 befestigt sind (nicht sichtbar in Figur 2a). Ein Permanentmagnet 40 des Positionssensors 38 ist am Bewegungselement 24 befestigt.

Figur 2b zeigt den Positionssensor im Detail. Der Positionssensor 38 umfasst den Permanentmagneten 40 und die Hall-Sensoren 42.i (i = 1, 2, ..., 18) sowie die Auswerteeinrichtung 34. Die Hall-Sensoren 42.i sind am Bezugselement 26, im vorliegenden Fall also starr relativ zum Schaft 12, befestigt. Der Permanentmagnet 40 hingegen ist am Bewegungselement 24, im vorliegenden Fall also starr relativ zum Unterschenkel 16, befestigt.

Bewegt sich das Bewegungselement 24 relativ zum Bezugselement 26, so bewegen sich die Hall-Sensoren 42.i relativ zum Bewegungselement 24 auf einer Trajektorie T. Im vorliegenden Fall ist die Trajektorie T ein Kreisbogen. Im vorliegenden Fall hängt die Trajektorie T vom Schwenkwinkel $\alpha$ (vgl. Figur 1) zwischen dem Bewegungselement 24 und dem Bezugselement 26 ab.

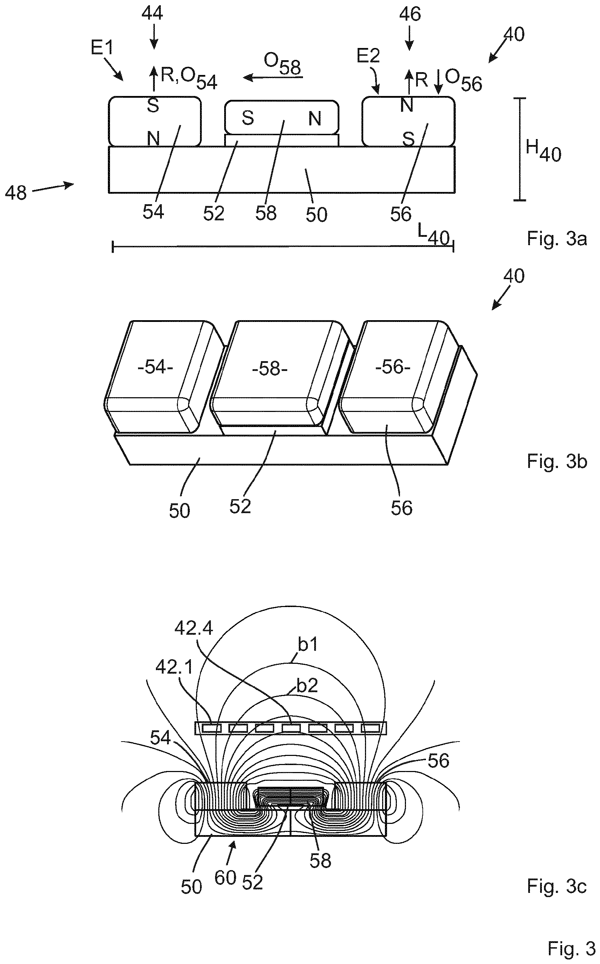

Figur 3a zeigt eine seitliche Ansicht des Permanentmagneten 40. Der Permanentmagnet 40 besitzt einen ersten Magnetschenkel 44, der sich in eine Magnetschenkel-Richtung R erstreckt. Der Permanentmagnet 40 besitzt zudem einen zweiten Magnetschenkel 46, der sich ebenfalls entlang der Magnetschenkel-Richtung R erstreckt. Der erste Magnetschenkel 44 hat ein erstes freies Ende E1 mit einer ersten Polarität P1, die im vorliegenden Fall der Südpol ist. Der zweite Magnetschenkel 46 hat ein zweites freies Ende E2, das eine der ersten Polarität P1 entgegengesetzte zweite Polarität P2 hat, im vorliegenden Fall also ein Nordpol ist.

Der Permanentmagnet 40 besitzt zudem ein Magnetflussformteil 48, das ein Weichmagnet-Element 50 und einen Nichtferromagnetteil 52 aufweist. Das Weichmagnet-Element 50 besteht in der vorliegenden Ausführungsform aus Weicheisen, das Nichtferromagnetteil 52 im vorliegenden Fall aus Kupfer. Das Nichtferromagnetteil 52 könnte beispielsweise auch aus Kunststoff gefertigt sein und dient insbesondere als Abstandshalter.

Figur 3a zeigt, dass der Permanentmagnet 40 einen ersten Teil-Permanentmagneten 54, einen zweiten Teil-Permanentmagneten 56 und einen dritten Teil-Permanentmagneten 58 aufweist. Der erste Teil-Permanentmagnet 54 bildet den ersten Magnetschenkel 44, der zweite Teil-Permanentmagnet 56 den zweiten Magnetschenkel 46.

Der dritte Teil-Permanentmagnet 58 ist zwischen dem ersten Teil-Permanentmagneten 54 und dem zweiten Teil-Permanentmagneten 56 angeordnet und verläuft quer dazu. In anderen Worten verläuft eine Drittpermanentmagnet-Orientierung $O_{58}$, die vom Nordpol zum Südpol verläuft, quer zu einer Erstpermanentmagnet-Orientierung $O_{54}$, die im vorzugsweisen Fall der Magnetschenkel-Richtung R entspricht. Die dritte Permanentmagnet-Orientierung $O_{58}$ verläuft zudem quer zu einer Zweitpermanentmagnet-Orientierung $O_{56}$, die im vorliegenden Fall entgegen der Magnetschenkel-Richtung R verläuft. Unter dem Merkmal, dass die dritte Permanentmagnet-Orientierung $O_{58}$ quer zur ersten Permanentmagnet-Orientierung verläuft, wird insbesondere verstanden, dass ein Winkel zwischen beiden zumindest im Wesentlichen 90° beträgt. Das heißt, dass der Winkel zwischen 85 und 95° liegt.

Figur 3a zeigt, dass eine Höhe $H_{40}$ des Permanentmagneten 40 kleiner ist als seine Länge $L_{40}$. Die Höhe $H_{40}$ wird in Richtung der Magnetschenkel-Richtung R gemessen. Vorzugsweise ist die Länge $L_{40}$ zumindest doppelt so groß wie die Höhe $H_{40}$, vorzugsweise zumindest 2,5-fach so groß.

Figur 3b zeigt eine perspektivische Ansicht des Permanentmagneten 40. Die Teil-Permanentmagneten 54, 56, 58 und das Magnetflussformteil 48 sind miteinander verbunden, beispielsweise miteinander verklebt.

Figur 3c zeigt einen Magnetflusslinienverlauf 60 der Magnetflusslinien b1, b2, .... Es ist zu erkennen, dass eine Normalkomponente $B_N$ des Magnetfelds am Ort des Hall-Sensors 42.4, der sich exakt zwischen den beiden Enden E1 und E2 befindet, verschwindet.

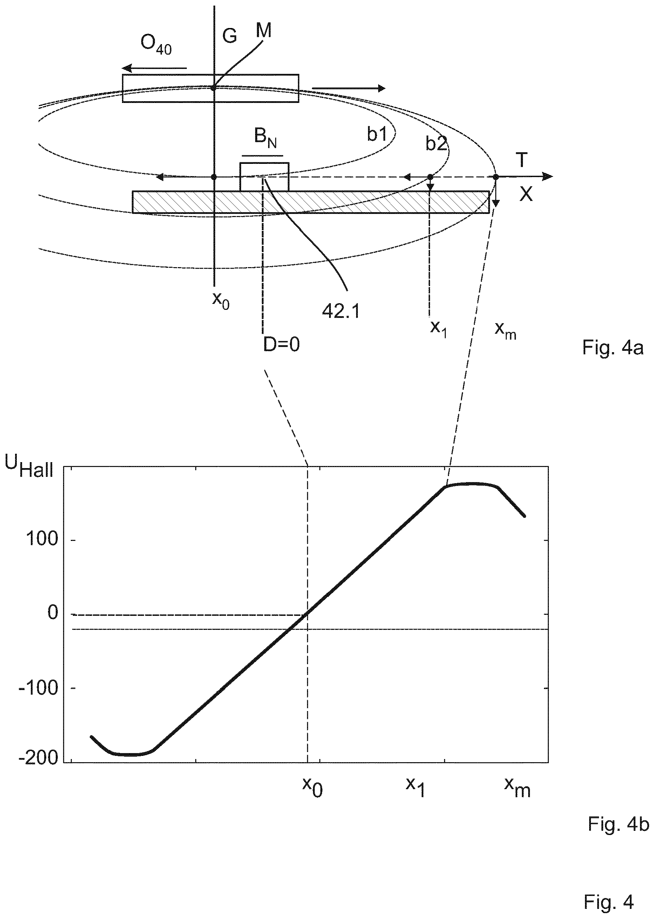

Figur 4a zeigt den Verlauf der Magnetflusslinien b1, b2, ..., wenn statt des Permanentmagneten, wie er in den Figuren 3a, 3b und 3c gezeigt ist, als Permanentmagnet ein Stabmagnet verwendet wird. Jeder Hall-Sensor 42, beispielsweise der Hall-Sensor 42.1, gibt eine in Figur 4b gezeigte Hall-Spannung $U_{Hall}$ ab, die von der Normalkomponente $B_N$ des Magnetfelds B in Bezug auf eine Sensorebene E des Hall-Sensor 42.1 abhängt. Auf einer Geraden G, die senkrecht zur Magnet-Orientierung $O_{40}$ durch einen Mittelpunkt M des Permanentmagneten 40 verläuft, existiert keine Normalkomponente $B_N$. Entsprechend ist die Hall-Spannung $U_{Hall}$ an dieser Stelle, die als $x_0$ bezeichnet wird, gleich null.

Wird der Permanentmagneten 40, der am Bewegungselement befestigt ist, bewegt, so bewegt sich der Hall-Sensor 42.1 entlang der Trajektorie T, die im vorliegenden Fall eine Gerade ist, relativ zum Bewegungselement. Der Permanentmagnet 40 bewegt sich damit relativ zum Bezugselement auch entlang der Trajektorie T. Dadurch ändert sich die Hall-Spannung $U_{Hall}$ zunächst linear und durchläuft in einem Punkt $x_M$ ein Maximum. Der Grund dafür ist, dass zwar einerseits der Winkel, den die Magnetfeldlinie mit der Sensorebene bildet, beständig größer wird, dass aber das Magnetfeld in dritter Potenz mit dem Abstand kleiner wird.

Figur 4b zeigt die Abhängigkeit der Hall-Spannung $U_{Hall}$ von der Position des Hall-Sensors.

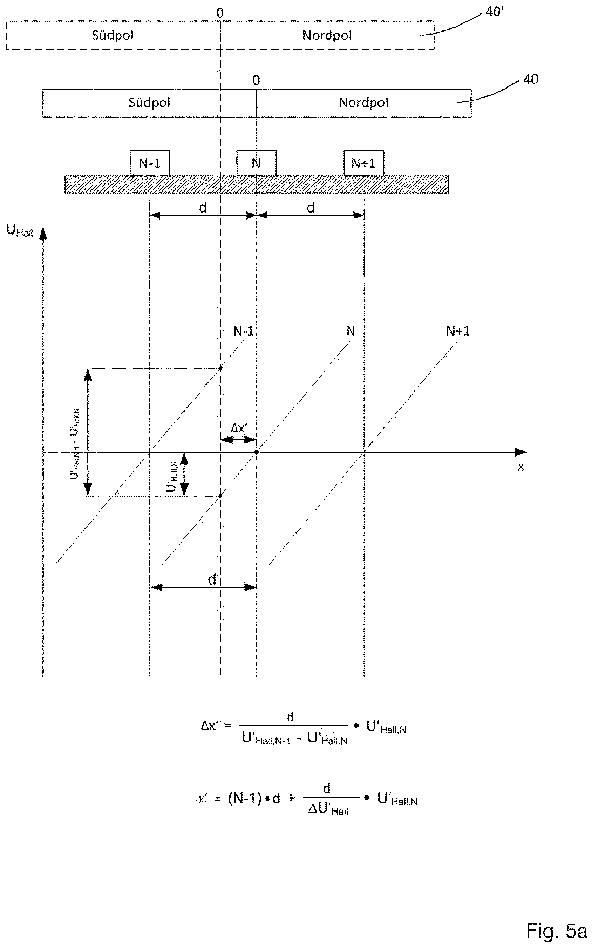

Figur 5a zeigt die Abhängigkeit der Hall-Spannung $U_{Hall}$ von einer x-Koordinate entlang der Trajektorie T. Im oberen Teil des Bildes sind die Positionen der allgemein bezeichneten Hall-Sensoren 42.N, 42.N+1 und 42.N-1 eingezeichnet. Im unteren Teilbild ist zu erkennen, dass die Steigung $k = \Delta U_{Hall}/d$ bestimmt werden kann als

$$k := \frac{U_{Hall,N-1} - U_{Hall,N}}{d} = \frac{\Delta U_{Hall}}{d}$$

d ist der Abstand zweier Hall-Sensoren, beispielsweise der Hall-Sensoren 42.N und 42.N+1. $\Delta U_{Hall}$ ist die Hallspannungs-Differenz. Die Hall-Sensoren 42.i sind äquidistant angeordnet, das heißt, dass der Abstand zweier benachbarter Hall-Sensoren stets gleich d ist.

Wird der Permanentmagnet verschoben, beispielsweise an die gestrichelt gezeichnete Position, so verschiebt sich der Spannungsverlauf ebenfalls. Aus der Verschiebung $\Delta x'$ entlang der Trajektorie T ergibt sich die Hallspannung $U'_{Hall,N}$, die der Hall-Sensor 42.N nach der Verschiebung um $\Delta x'$ misst zu

$$U'_{Hall,N} = k\Delta x' = \frac{\Delta U'_{Hall}}{d} \Delta x'$$

Damit kann aus der jeweils gemessenen Hall-Spannung $U'_{Hall,N}$ die Position des Hall-Sensors 42.N und damit die Position des Bezugselements 26 relativ zum Bewegungselement 24 (vgl. Figur 2) bestimmt werden. Es existieren in der Regel mehr als drei Hall-Sensoren 42.i.

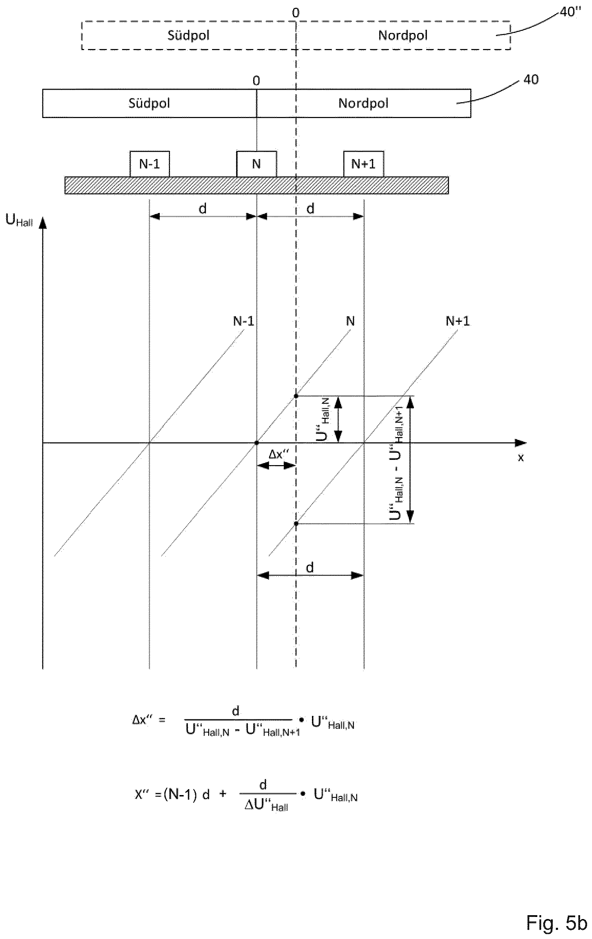

In Figur 5b sind die Abhängigkeiten der Hall-Spannung $U_{Hall}$, i für mehrere Hall-Sensoren dargestellt. Befindet sich der Permanentmagnet 40 in der Position $\Delta x''$ relativ zu den Hall-Sensoren, wie in Figur 5b gestrichelt angedeutet (und mit Permanentmagnet 40" bezeichnet), so werden die angegebenen Hall-Spannungen $U''_{Hall,N-1},\ldots, U''_{Hall,N+2}$ gemessen.

In einem ersten Schritt werden diejenigen Hall-Spannungen bestimmt, die am dichtesten an demjenigen Wert liegen, der an der Position gemessen wird, die in Figur 5b für den Hall-Sensor 42.n eingezeichnet ist, nämlich in der der Hall-Sensor genau zwischen den beiden magnetischen Polen des Permanentmagneten 40 angeordnet ist. Dieser Wert ist in aller Regel $U''_{Hall} = 0$ V, da dann keine Normal-Magnetfeldkomponente für den entsprechenden Hall-Sensor existiert.

Im vorliegenden Fall werden also die drei betragsmäßig kleinsten Hall-Spannungen $U''_{Hall}$ bestimmt. Das sind die Hall-Spannungen $U''_{Hall,N-1}, \ldots , U''_{Hall,N+1}$. Der betragsmäßig kleinste Wert ist $U''_{Hall,N}$. Die betragsmäßig nächstkleinere Hall-Spannung ist $U_{Hall,N+1}$.

Es gilt daher

$$U''_{Hall,N} = +k\Delta x''.$$

Die Position ergibt sich damit zu

$$x'' = (N - 1)d + \frac{\Delta U''_{Hall}}{k}.$$

In dieser Formel ist x" der Weg entlang der Trajektorie T, wobei x = 0 an der Stelle des ersten Hall-Sensors 42.1 gilt.

Bewegt sich der Permanentmagnet 40 weiter, so wird beispielsweise die Spannung $U''_{Hall,N}$ immer größer, bis sie betragsmäßig größer wird als die Spannung $U''_{Hall,N+1}$. Zu diesem Zeitpunkt wird die Berechnung mit der dann betragsmäßig kleinsten Hall-Spannung durchgeführt. Es sei darauf hingewiesen, dass sich die betragsmäßig kleinsten Spannungen stets auf diejenige Spannung beziehen, die der Hall-Sensor hat, der wie der Hall-Sensor 42.N in Figur 5a angeordnet ist, also in dem Bereich genau zwischen den beiden freien Enden des Permanentmagneten 40. Diese Spannung ist in der Regel null.

Es ist möglich, dass beispielsweise durch eine verkippte Montage der Hall-Sensoren, diese Spannung nicht null ist, sondern eine Offset-Spannung $U_{Offset}$. In diesem Fall wird die jeweils gemessene Hall-Spannung $U_{Hall, mess}$ um die Offset-Spannung $U_{Offset}$ korrigiert. Die Offsetspannungen $U_{Offset}$ der Hall-Sensoren wird in einem Kalibriervorgang gemessen, wenn der Magnet nicht in der Nähe der Hall-Sensoren ist. Die gemessenen Hall-Spannungen $U_{Hall, mess}$ werden bei der Auswertung um den Wert der Offset-Spannung korrigiert. Die oben angegebenen Hall-Spannungen $U'_{Hall,N}$ entsprechen dem korrigierten Wert der Hall Spannungen. Es ergibt sich dann wieder die in Figur 5 gezeigte Situation, dass die Hall-Spannung null ist, wenn sich der entsprechende Hall-Sensor genau zwischen den freien Enden des Permanentmagneten befindet.

Es ist möglich und stellt eine bevorzugte Ausführungsform dar, dass solche Hall-Sensoren, die momentan nicht zur Bestimmung der Position notwendig sind, deaktiviert werden. In anderen Worten wird die Messung der Hall-Spannung von der Auswerteeinheit 34 solange beendet, bis der Messwert des entsprechenden Hall-Sensors wieder benötigt wird. Dazu ist es möglich, dass die Hall-Sensoren in Gruppen eingeteilt werden. Wir kein Hall-Sensor einer entsprechenden Gruppe verwendet, werden die Hall-Sensoren der entsprechenden Gruppe abgeschaltet.

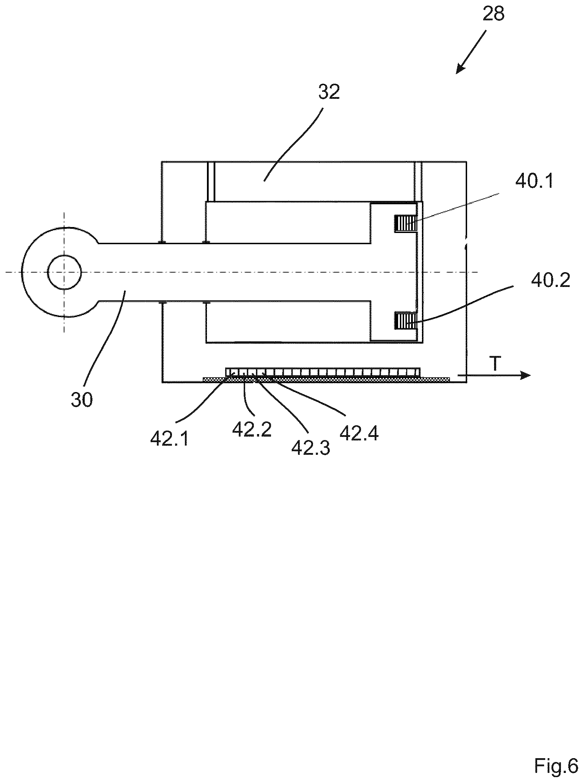

Figur 6 zeigt den Dämpfer 28. Es ist zu erkennen, dass am Kolben 30 zwei Permanentmagnete 40.1, 40.2 angeordnet sind. Die Hall-Sensoren 42.i sind linear angeordnet. Bei einer Bewegung des Kolbens 30 relativ zum Zylinder 32 und damit einer Bewegung des Zylinders 32 relativ zum Kolben 30 bewegen sich die Hall-Sensoren entlang einer Trajektorie T relativ zu den Permanentmagneten 40.1, 40.2. Auf die oben beschriebene Art und Weise kann die Position des Kolbens 30 relativ zum Zylinder 32 mit relativ hoher Genauigkeit abgestimmt werden. Bei der Ausführungsform gemäß Figur 1 wird eine Winkelauflösung von 0,01 Grad erreicht. Bei der Ausführungsform gemäß Figur 6 ergibt sich eine Genauigkeit von ca. 0,1 mm.

**Bezugszeichenliste**

| | | b | Magnetflusslinie |
|---|---|---|---|
| 10 | Hilfsmittel | $B_N$ | Normalkomponente |
| 12 | Schaft | d | Abstand |
| 14 | Oberschenkelstumpf | E | Sensorebene |

(fortgesetzt)

| | | | |
|---|---|---|---|
| 16 | Unterschenkel | E1 | erstes freies Ende |
| | | E2 | zweites freies Ende |
| 20 | Fuß | G | Gerade |
| 22 | Kosmetik | H | Höhe |
| 22 | Drehgelenk | i | Laufindex |
| 24 | Bewegungselement | L | Länge |
| 26 | Bezugselement | M | Mittelpunkt |
| 28 | Dämpfer | $O_{54}$ | Erstpermanentmagnet-Orientierung |
| 30 | Kolben | | |
| 32 | Zylinder | $O_{56}$ | Zweitpermanentmagnet-Orientierung |
| 34 | Auswerteeinheit | | |
| 36 | Aktor | Oss | Drittpermanentmagnet-Orientierung |
| 38 | Positionssensor | | |
| | | P | Polarität |
| 40 | Permanentmagnet | R | Magnetschenkel-Richtung |
| 42 | Hall-Sensor | T | Trajektorie |
| 44 | erster Magnetschenkel | $U_{Hall}$ | Hall-Spannung |
| 46 | zweiter Magnetschenkel | $\Delta U_{Hall}$ | Hallspannungs-Differenz |
| 48 | Magnetflussformteil | $\alpha$ | Schwenkwinkel |
| 50 | Weichmagnet-Element | | |
| 52 | Nichtferromagnetteil | | |
| 54 | erster Teil-Permanentmagnet | | |
| 56 | zweiter Teil-Permanentmagnet | | |
| 58 | dritter Teil-Permanentmagnet | | |
| 60 | Magnetflusslinienverlauf | | |

**Patentansprüche**

1. Orthopädisches Hilfsmittel (10) mit

   (a) einem Bezugselement (26),
   (b) einem Bewegungselement (24), das am Bezugselement (26) bewegbar befestigt ist, und
   (c) einem Positionssensor (38) zum Bestimmen einer Position des Bewegungselements (24) relativ zum Bezugselement (26), der

      - zumindest einen Permanentmagneten (40) und
      - zumindest drei Hall-Sensoren (42) aufweist,

   (d) wobei der zumindest eine Permanentmagnet (40) am Bewegungselement (24) befestigt ist,

   **dadurch gekennzeichnet, dass**

      (e) die Hall-Sensoren (42) am Bezugselement (26), und zum Bewegen entlang einer Trajektorie (T) beim Bewegen des Bewegungselements (24) relativ zum Bezugselement (26), angeordnet sind und
      (f) die Hall-Sensoren (42) und der Permanentmagnet (40) so angeordnet sind, dass eine Bewegung des Bewegungselements (24) relativ zum Bezugselement (26) für zumindest einen Hall-Sensor (42) eine Änderung seiner Hall-Spannung ($U_{Hall}$) bewirkt, die linear von der Position entlang der Trajektorie abhängt.

2. Orthopädisches Hilfsmittel (10) nach Anspruch 1, **dadurch gekennzeichnet, dass**

   (a) der zumindest eine Permanentmagnet (40)

      - einen ersten Magnetschenkel (44), der sich in eine Magnetschenkel-Richtung (R) erstreckt und

- einen zweiten Magnetschenkel (46), der vom ersten Magnetschenkel (44) beabstandet ist und sich entlang der Magnetschenkel-Richtung (R) erstreckt, aufweist,

(b) der erste Magnetschenkel (44) ein erstes freies Ende (E1) hat, das eine erste Polarität (P1) hat,
(c) der zweite Magnetschenkel (46) ein zweites freies Ende (E2) hat, das eine zweite Polarität (P2) hat, die der ersten Polarität (P1) entgegengesetzt ist, und dass
(d) die freien Enden (E1, E2) entlang der Trajektorie (T) angeordnet sind.

3. Orthopädisches Hilfsmittel (10) nach Anspruch 2, **dadurch gekennzeichnet, dass** der Permanentmagnet (40)

(a) ein Magnetflussformteil (48),
das ein Weichmagnet-Element (50), das aus einem weichmagnetischen Werkstoff besteht,
(b) einen ersten Teil-Permanentmagneten (54), der

- den ersten Magnetschenkel (44) bildet und
- mit seinem dem ersten freien Ende (E1) gegenüberliegenden ersten Kontakt-Ende am Weichmagnet-Element (50) anliegt,

(c) einen zweiten Teil-Permanentmagneten (56), der

- den zweiten Magnetschenkel (46) bildet und
- mit seinem dem zweiten freien Ende (E2) gegenüberliegenden zweiten Kontakt-Ende am Weichmagnet-Element (50) anliegt, und

(d) einen dritten Teil-Permanentmagneten (48), der

- zwischen dem ersten Teil-Permanentmagneten (54) und dem zweiten Teil-Permanentmagneten (56) angeordnet ist,
- quer zum ersten Magnetschenkel (44) und zum zweiten Magnetschenkel (46) verläuft, aufweist und
- eine magnetische Drittpermanentmagnet-Orientierung ($O_{58}$) hat, die quer zu einer Erstpermanentmagnet-Orientierung ($O_{54}$) des ersten Teil-Permanentmagneten (54) verläuft und quer zu einer Zweitpermanentmagnet-Orientierung ($O_{55}$) des zweiten Teil-Permanentmagneten (56) verläuft.

4. Orthopädisches Hilfsmittel (10) nach einem der vorstehenden Ansprüche 2 oder 3, **dadurch gekennzeichnet, dass** das Magnetflussformteil (48) ein Nichtferromagnetteil (52) aufweist, das im Magnetflusslinienverlauf (60) zwischen dem ersten Teil-Permanentmagneten (54) und dem dritten Teil-Permanentmagneten (58), und/oder im Magnetflusslinienverlauf (60) zwischen dem zweiten Teil-Permanentmagneten (56) und dem dritten Teil-Permanentmagneten (58) angeordnet ist.

5. Orthopädisches Hilfsmittel (10) nach einem der vorstehenden Ansprüche, **dadurch gekennzeichnet, dass** der Permanentmagnet (40) eine Permanentmagnet-Länge ($L_{40}$) entlang der Trajektorie (T) hat, die zumindest doppelt, insbesondere zumindest dreifach, so groß ist wie ein Hallsensoren-Abstand (d) zweier benachbarter Hall-Sensoren (42).

6. Orthopädisches Hilfsmittel (10) nach einem der vorstehenden Ansprüche, **dadurch gekennzeichnet, dass** ein Abstand der Hall-Sensoren (42) vom Permanentmagneten (40) höchstens die Hälfte, vorzugsweise höchstens ein Drittel, der Permanentmagnet-Länge ($L_{40}$) beträgt.

7. Orthopädisches Hilfsmittel (10) nach einem der vorstehenden Ansprüche, **gekennzeichnet durch** eine elektrische Auswerteeinheit (34), die ausgebildet ist zum automatischen Durchführen eines Verfahrens mit den Schritten:

(i) Erfassen einer ersten Hall-Spannung ($U_{Hall,N-1}$) eines ersten Hall-Sensors (42.N-1) und einer zweiten Hall-Spannung ($U_{Hall,N}$) eines zum ersten Hall-Sensors (42.N-1) benachbarten zweiten Hall-Sensors (42.N) und
(ii) Bestimmen einer Position des Bewegungselements (24) relativ zum Bezugselement (26) aus einer Hallspannungs-Differenz ($\Delta U_{Hall}$), die aus erster Hall-Spannung ($U_{Hall,N-1}$) und zweiter Hall-Spannung ($U_{Hall,N}$) gebildet ist, und der zweiten Hall-Spannung ($U_{Hall,N}$).

8. Orthopädisches Hilfsmittel (10) nach einem der vorstehenden Ansprüche, **gekennzeichnet durch** eine elektrische Auswerteeinheit (34), die ausgebildet ist zum automatischen Durchführen eines Verfahrens mit den Schritten:

(i) Erfassen der Hall-Spannungen ($U_{Hall,i}$) von zumindest drei Hall-Sensoren (42),

(ii) Erfassen derjenigen zwei Hall-Sensoren (42), bei denen die Hall-Spannungen ($U_{Hall}$) die betragsmäßig kleinsten Werte annehmen, und

(iii) Bestimmen der Position des Bewegungselements (24) relativ zum Bezugselement (26) aus den Positionen dieser Hall-Sensoren (42) und einer Hall-Spannungs-Differenz dieser Hall-Spannungen ($U_{Hall}$).

9. Orthopädisches Hilfsmittel (10) nach einem der vorstehenden Ansprüche, **dadurch gekennzeichnet, dass**

(a) das Bezugselement (26) ein Zylinder (32) ist,

(b) das Bewegungselement (24) ein Kolben (30) ist, der im Zylinder (32) läuft,

(c) der Positionssensor ein Kolben-Positionssensor zum Messen einer Position des Kolbens (30) im Zylinder (32),

(d) der Permanentmagnet (40) am Kolben (30) angeordnet ist und

(e) die Hall-Sensoren (42) am Zylinder (32) angeordnet sind.

10. Orthopädisches Hilfsmittel (10) nach einem der vorstehenden Ansprüche, **dadurch gekennzeichnet, dass**

(a) das Bezugselement (26) ein erster Schenkel ist,

(b) das Bewegungselement (24) ein zweiter Schenkel ist und dass

(c) der Positionssensor (38) ein Schenkel-Winkelsensor zum Messen einer Winkelstellung des ersten Schenkels relativ zum zweiten Schenkel ist.

11. Orthopädisches Hilfsmittel (10) nach einem der vorstehenden Ansprüche, **dadurch gekennzeichnet, dass** die Auswerteeinheit (34) ausgebildet ist zum Abschalten von solchen Hall-Sensoren (42), deren Messergebnisse nicht an der Berechnung der Position des Bewegungselements (24) beteiligt sind.

12. Verfahren zum Bestimmen einer Stellung eines orthopädischen Hilfsmittels (10) gemäß einem der vorstehenden Ansprüche mit den Schritten:

(i) Erfassen einer ersten Hall-Spannung ($U_{Hall,N-1}$) eines ersten Hall-Sensors (42.N-1) und einer zweiten Hall-Spannung ($U_{Hall,N}$) eines zum ersten Hall-Sensor (42.N-1) benachbarten zweiten Hall-Sensors (42.N) und

(ii) Bestimmen der Position des Bewegungselements (24) relativ zum Bezugselement (26) aus einer Hallspannungs-Differenz ($\Delta U_{Hall}$), die aus erster Hall-Spannung ($U_{Hall,N-1}$) und zweiter Hall-Spannung ($U_{Hall,N}$) gebildet ist, und der zweiten Hall-Spannung ($U_{Hall,N}$).

13. Verfahren nach Anspruch 12, **gekennzeichnet durch** die Schritte:

(i) Erfassen der Hall-Spannungen ($U_{Hall, N-1}$, $U_{Hall, N}$ $U_{Hall, N+1}$) von zumindest drei Hall-Sensoren (42),

(ii) Erfassen derjenigen zwei Hall-Sensoren (42), bei denen die Hall-Spannungen ($U_{Hall, N-1}$, $U_{Hall, N}$ $U_{Hall, N+1}$) die betragsmäßig kleinsten Werte annehmen, und

(iii) Bestimmen der Position des Permanentmagneten (40) aus den Positionen dieser Hall-Sensoren (42) und einer Hallspannungs-Differenz ($\Delta U_{Hall}$), die aus diesen zwei Hall-Spannungen ($U_{Hall, N-1}$, $U_{Hall, N}$ $U_{Hall, N+1}$) gebildet ist, und der Hall-Spannung ($U_{Hall,N}$).

14. Verfahren nach Anspruch 12 oder 13, **gekennzeichnet durch** die Schritte:

(i) für jeden Hall-Sensor (42) Erfassen einer Offset-Spannung ($U_{Offset}$) der Hall-Spannung ($U_{Hall}$) aufgrund eines Winkels zwischen einer Ist-Lage des Hall-Sensors und (42) einer Soll-Lage und

(ii) Korrigieren der Hall-Spannung ($U_{Hall}$) um die Offset-Spannung ($U_{Offset}$).

15. Verfahren nach Anspruch 12 bis 14, **gekennzeichnet durch** die Schritte:

(i) für jeden Hall-Sensor (42) Erfassen einer Sensitivität, die die Abhängigkeit der Hall-Spannung vom Magnetfeld beschreibt, und

(ii) Korrigieren der Position um den Einfluss der Sensitivität.

PI/bro-be, cba

**Claims**

1.  An orthopedic aid (10) with

    (a) a reference element (26),
    (b) a movement element (24) that is movably fixed to the reference element (26), and
    (c) a position sensor (38) for determining a position of the movement element (24) relative to the reference element (26) which comprises

    - at least one permanent magnet (40) and
    - at least three Hall sensors (42),

    (d) the at least one permanent magnet (40) being fixed to the movement element (24),

    **characterised in that**

    (e) the Hall sensors (42) are arranged on the reference element (26) and are arranged to move along a trajectory (T) when the movement element (24) moves relative to the reference element (26) and
    (f) the Hall sensors (42) and the permanent magnet (40) are arranged in such a way that a movement of the movement element (24) relative to the reference element (26) effects a change in the Hall voltage ($U_{Hall}$) for at least one Hall sensor (42), said voltage being linearly dependent on the position along the trajectory.

2.  The orthopedic aid (10) according to claim 1, **characterised in that**

    (a) the at least one permanent magnet (40) has

    - a first magnetic leg (44) that extends in a magnetic leg direction (R) and
    - a second magnetic leg (46) that is spaced apart from the first magnetic leg (44) and extends along the magnetic leg direction (R),

    (b) the first magnetic leg (44) has a first free end (E1), which has a first polarity (P1),
    (c) the second magnetic leg (46) has a second free end (E2) that has a second polarity (P2) opposite to the first polarity (P1), and that
    (d) the free ends (E1, E2) are arranged along the trajectory (T).

3.  The orthopedic aid (10) according to claim 2, **characterised in that** the permanent magnet (40) comprises

    (a) a magnetic flux moulded part (48),
    which has a soft magnet element (50) composed of a soft magnetic material,
    (b) a first partial permanent magnet (54) which

    - forms the first magnetic leg (44) and
    - rests with its first contact end opposite the first free end (E1) on the soft magnet element (50),

    (c) a second partial permanent magnet (56) which

    - forms the second magnetic leg (46) and
    - rests with its second contact end opposite the second free end (E2) on the soft magnet element (50), and

    (d) a third partial permanent magnet (48) which

    - is arranged between the first partial permanent magnet (54) and the second partial permanent magnet (56),
    - extends transversely to the first magnetic leg (44) and the second magnetic leg (46), and
    - has a magnetic third permanent magnet orientation (Oss) that extends transversely to a first permanent magnet orientation ($O_{54}$) of the first partial permanent magnet (54) and extends transversely to a second

permanent magnet orientation orientation ($O_{56}$) of the second partial permanent magnet (56).

4. The orthopedic aid (10) according to one of the claims 2 or 3, **characterised in that**
the magnetic flux moulded part (48) comprises a non-ferromagnetic part (52) arranged in the magnetic flux line (60) between the first part permanent magnet (54) and the third part permanent magnet (58), and/or in the magnetic flux line (60) between the second part permanent magnet (56) and the third part permanent magnet (58).

5. The orthopedic aid (10) according to one of the preceding claims, **characterised in that**
the permanent magnet (40) has a permanent magnet length ($L_{40}$) along the trajectory (T) that is at least twice as large, particularly at least three times as large, as a Hall sensor distance (d) of two adjacent Hall sensors (42).

6. The orthopedic aid (10) according to one of the preceding claims, **characterised in that** a distance of the Hall sensors (42) from the permanent magnet (40) is at most half, preferably at most one third, of the permanent magnet length ($L_{40}$).

7. The orthopedic aid (10) according to one of the preceding claims, **characterised by** an electrical evaluation unit (34), which is configured to automatically carry out a method featuring the steps:

(i) detecting a first Hall voltage ($U_{Hall,N-1}$) of a first Hall sensor (42.N-1) and a second Hall voltage ($U_{Hall,N}$) of a second Hall sensor (42.N) adjacent to the first Hall sensor (42.N-1) and
(ii) determining a position of the movement element (24) relative to the reference element (26) from a Hall voltage difference ($\Delta U_{Hall}$), composed of the first Hall voltage ($U_{Hall,N-1}$) and the second Hall voltage ($U_{Hall,N}$), and the second Hall voltage ($U_{Hall,N}$).

8. The orthopedic aid (10) according to one of the preceding claims, **characterised by** an electrical evaluation unit (34), which is configured to automatically carry out a method featuring the steps::

(i) detecting the Hall voltages ($U_{Hall,i}$) of at least three Hall sensors (42),
(ii) detecting the two Hall sensors (42) for which the Hall voltages ($U_{Hall}$) assume the smallest values in terms of magnitude and
(iii) determinig the position of the movement element (24) relative to the reference element (26) from the positions of these Hall sensors (42) and a Hall voltage difference of these Hall voltages ($U_{Hall}$).

9. The orthopedic aid (10) according to one of the preceding claims, **characterised in that**

(a) the reference element (26) is a cylinder (32),
(b) the movement element (24) is a piston (30) that extends in the cylinder (32),
(c) the position sensor is a piston position sensor for measuring a position of the piston (30) in the cylinder (32),
(d) the permanent magnet (40) is arranged on the piston (30) and
(e) the Hall sensors (42) are arranged on the cylinder (32).

10. The orthopedic aid (10) according to one of the preceding claims, **characterised in that**

(a) the reference element (26) is a first leg,
(b) the movement element (24) is a second leg, and **in that**
(c) the position sensor (38) is a leg angle sensor for measuring an angular position of the first leg relative to the second leg.

11. The orthopedic aid (10) according to one of the preceding claims, **characterised in that** the evaluation unit (34) is configured to switch off Hall sensors (42) whose measurement results are not included in the calculation of the position of the movement element (24).

12. A method for determining a position of an orthopedic aid (10) according to one of the preceding claims featuring the steps:

(i) detecting a first Hall voltage ($U_{Hall,N-1}$) of a first Hall sensor (42.N-1) and a second Hall voltage ($U_{Hall,N}$) of a second Hall sensor (42.N) adjacent to the first Hall sensor (42.N-1) and
(ii) determining the position of the movement element (24) relative to the reference element (26) from a Hall

voltage difference ($\Delta U_{Hall}$), composed of the first Hall voltage ($U_{Hall,N-1}$) and the second Hall voltage ($U_{Hall,N}$), and the second Hall voltage ($U_{Hall,N}$).

13. The method according to claim 12, **characterised by** the steps:

(i) detecting the Hall voltages ($U_{Hall, N-1}$, $U_{Hall, N}$ $U_{Hall, N+1}$) of at least three Hall sensors (42),
(ii) detecting the Hall sensors (42) for which the Hall voltages ($U_{Hall, N-1}$, $U_{Hall, N}$ $U_{Hall, N+1}$) assume the smallest values in terms of magnitude, and
(iii) determining the position of the permanent magnet (40) from the positions of these Hall sensors and a Hall voltage difference ($\Delta U_{Hall}$), composed of these two Hall voltages ($U_{Hall, N-1}$, $U_{Hall, N}$ $U_{Hall, N+1}$), and the Hall voltage ($U_{Hall,N}$).

14. The method according to claim 12 or 13, **characterised by** the steps:

(i) for each Hall sensor (42), detecting an offset voltage ($U_{offset}$) of the Hall voltage ($U_{Hall}$) due to an angle between an actual position of the Hall sensor (42) and a target position, and
(ii) correcting the Hall voltage ($U_{Hall}$) by the offset voltage (Uoffset).

15. The method according to claim 12 to 14, **characterised by** the steps:

(i) for each Hall sensor (42), detecting a sensitivity that denotes the dependence of the Hall voltage on the magnetic field, and
(ii) correcting the position by the influence of the sensitivity.

PI/bro-be, cba

**Revendications**

1. Moyen auxiliaire orthopédique (10) comprenant

(a) un élément de référence (26),
(b) un élément de mouvement (24) fixé de manière mobile à l'élément de référence (26), et
(c) un capteur de position (38) pour déterminer une position de l'élément de mouvement (24) par rapport à l'élément de référence (26), qui comprend

- au moins un aimant permanent (40) et
- au moins trois capteurs à effet Hall (42),

(d) ledit au moins un aimant permanent (40) étant fixé à l'élément de mouvement (24),

**caractérisé en ce que**

(e) les capteurs à effet Hall (42) sont disposés sur l'élément de référence (26) et destinés à se déplacer le long d'une trajectoire (T) lorsque l'élément de mouvement (24) se déplace par rapport à l'élément de référence (26), et
(f) les capteurs à effet Hall (42) et l'aimant permanent (40) sont disposés de telle sorte qu'un mouvement de l'élément de mouvement (24) par rapport à l'élément de référence (26) provoque, pour au moins un capteur à effet Hall (42), une modification de sa tension Hall ($U_{Hall}$) qui dépend linéairement de la position le long de la trajectoire.

2. Moyen auxiliaire orthopédique (10) selon la revendication 1, **caractérisé en ce que**

(a) ledit au moins un aimant permanent (40) comprend

- une première branche magnétique (44) qui s'étend dans une direction de branche magnétique (R) et
- une deuxième branche magnétique (46) qui est espacée de la première branche magnétique (44) et qui s'étend le long de la direction de branche magnétique (R),

(b) la première branche magnétique (44) présente une première extrémité libre (E1) qui a une première polarité (P1),

(c) la deuxième branche magnétique (46) présente une deuxième extrémité libre (E2) qui a une deuxième polarité (P2) opposée à la première polarité (P1),

et **en ce que**

(d) les extrémités libres (E1, E2) sont disposées le long de la trajectoire (T).

3. Moyen auxiliaire orthopédique (10) selon la revendication 2, **caractérisé en ce que** l'aimant permanent (40) comprend

(a) une partie moulée de flux magnétique (48),
qui comprend un élément magnétique doux (50) réalisé en un matériau magnétique doux,
(b) un premier aimant permanent partiel (54) qui

- constitue la première branche magnétique (44) et
- s'appuie par sa première extrémité de contact, opposée à la première extrémité libre (E1), sur l'élément magnétique doux (50),

(c) un deuxième aimant permanent partiel (56) qui

- constitue la deuxième branche magnétique (46) et
- s'appuie par sa deuxième extrémité de contact, opposée à la deuxième extrémité libre (E2), sur l'élément magnétique doux (50), et

(d) un troisième aimant permanent partiel (48) qui

- est disposé entre le premier aimant permanent partiel (54) et le deuxième aimant permanent partiel (56),
- s'étend transversalement par rapport à la première branche magnétique (44) et à la deuxième branche magnétique (46), et
- présente une orientation magnétique de troisième aimant permanent ($O_{58}$) qui s'étend transversalement par rapport à une orientation de premier aimant permanent ($Osa$) du premier aimant permanent partiel (54) et qui s'étend transversalement par rapport à une orientation de deuxième aimant permanent ($O_{56}$) du deuxième aimant permanent partiel (56).

4. Moyen auxiliaire orthopédique (10) selon l'une des revendications 2 ou 3, **caractérisé en ce que** la partie moulée de flux magnétique (48) comprend une partie non ferromagnétique (52) qui est disposée dans le tracé des lignes de flux magnétique (60) entre le premier aimant permanent partiel (54) et le troisième aimant permanent partiel (58), et/ou dans le tracé des lignes de flux magnétique (60) entre le deuxième aimant permanent partiel (56) et le troisième aimant permanent partiel (58).

5. Moyen auxiliaire orthopédique (10) selon l'une des revendications précédentes, **caractérisé en ce que** l'aimant permanent (40) présente une longueur d'aimant permanent ($L_{40}$) le long de la trajectoire (T), qui est au moins deux fois, en particulier au moins trois fois, plus grande qu'une distance de capteur à effet Hall (d) de deux capteurs à effet Hall (42) adjacents.

6. Moyen auxiliaire orthopédique (10) selon l'une des revendications précédentes, **caractérisée en ce qu'**une distance entre les capteurs à effet Hall (42) et l'aimant permanent (40) est au plus égale à la moitié, de préférence au plus égale à un tiers, de la longueur de l'aimant permanent ($L_{40}$).

7. Moyen auxiliaire orthopédique (10) selon l'une des revendications précédentes, **caractérisé par** une unité d'évaluation électrique (34) qui est réalisée pour mettre en oeuvre automatiquement un procédé comprenant les étapes consistant à :

(i) détecter une première tension de Hall ($U_{Hall,N-1}$) d'un premier capteur à effet Hall (42.N-1) et une deuxième tension de Hall ($U_{Hall,N}$) d'un deuxième capteur à effet Hall (42.N) adjacent au premier capteur à effet Hall (42.N-1), et
(ii) déterminer une position de l'élément de mouvement (24) par rapport à l'élément de référence (26) à partir d'une différence de tension de Hall ($\Delta U_{Hall}$), formée entre la première tension de Hall ($U_{Hall,N-1}$) et la deuxième tension de Hall ($U_{Hall,N}$), et à partir de la deuxième tension de Hall ($U_{Hall,N}$).

8. Moyen auxiliaire orthopédique (10) selon l'une des revendications précédentes, **caractérisé par** une unité d'évaluation électrique (34) qui est réalisée pour mettre en oeuvre automatiquement un procédé comprenant les étapes consistant à :

(i) détecter les tensions de Hall ($U_{Hall,i}$) d'au moins trois capteurs à effet Hall (42),
(ii) détecter les deux capteurs à effet Hall (42) pour lesquels les tensions de et Hall ($U_{Hall}$) prennent les valeurs les plus faibles en valeur absolue, et
(iii) déterminer la position de l'élément de mouvement (24) par rapport à l'élément de référence (26) à partir des positions de ces capteurs à effet Hall (42) et d'une différence de tension de Hall entre ces tensions de Hall ($U_{Hall}$).

9. Moyen auxiliaire orthopédique (10) selon l'une des revendications précédentes, **caractérisé en ce que**

(a) l'élément de référence (26) est un cylindre (32),
(b) l'élément de mouvement (24) est un piston (30) qui se déplace dans le cylindre (32),
(c) le capteur de position est un capteur de position de piston pour mesurer une position du piston (30) dans le cylindre (32),
(d) l'aimant permanent (40) est disposé sur le piston (30), et
(e) les capteurs à effet Hall (42) sont disposés sur le cylindre (32).

10. Moyen auxiliaire orthopédique (10) selon l'une des revendications précédentes, **caractérisé en ce que**

(a) l'élément de référence (26) est une première branche,
(b) l'élément de mouvement (24) est une deuxième branche, et
(c) le capteur de position (38) est un capteur d'angle de branche pour mesurer une position angulaire de la première branche par rapport à la deuxième branche.

11. Moyen auxiliaire orthopédique (10) selon l'une des revendications précédentes, **caractérisée en ce que** l'unité d'évaluation (34) est réalisée pour déconnecter ceux des capteurs à effet Hall (42) dont les résultats de mesure ne sont pas impliqués dans le calcul de la position de l'élément de mouvement (24).

12. Procédé pour déterminer une position d'un moyen auxiliaire orthopédique (10) selon l'une des revendications précédentes, comprenant les étapes consistant à :

(i) détecter une première tension de Hall ($U_{Hall,N-1}$) d'un premier capteur à effet Hall (42.N-1) et une deuxième tension de Hall ($U_{Hall,N}$) d'un deuxième capteur à effet Hall (42.N) adjacent au premier capteur à effet Hall (42.N-1), et
(ii) déterminer la position de l'élément de mouvement (24) par rapport à l'élément de référence (26) à partir d'une différence de tension de Hall ($\Delta U_{Hall}$), formée entre la première tension de Hall ($U_{Hall,N-1}$) et la deuxième tension de Hall ($U_{Hall,N}$), et à partir de la deuxième tension de Hall ($U_{Hall,N}$).

13. Procédé selon la revendication 12, **caractérisé par** les étapes consistant à :

(i) détecter les tensions de Hall ($U_{Hall,N-1}$, $U_{Hall,N}$, $U_{Hall,N+1}$) d'au moins trois capteurs à effet Hall (42),
(ii) détecter les deux capteurs à effet Hall (42) pour lesquels les tensions de Hall ($U_{Hall,N-1}$, $U_{Hall,N}$, $U_{Hall,N+1}$) prennent les valeurs les plus faibles en valeur absolue, et
(iii) déterminer la position de l'aimant permanent (40) à partir des positions des ces capteurs à effet Hall (42) et d'une différence de tension de Hall ($\Delta U_{Hall}$), formée entre ces deux tensions de Hall ($U_{Hall,N-1}$, $U_{Hall,N}$, $U_{Hall,N+1}$), et à partir de la tension de Hall ($U_{Hall,N}$).

14. Procédé selon la revendication 12 ou 13, **caractérisé par** les étapes consistant à :

(i) pour chaque capteur à effet Hall (42), détecter une tension de décalage ($U_{offset}$) de la tension de Hall ($U_{Hall}$) en se basant sur un angle entre une position réelle du capteur à effet Hall (42) et une position de consigne, et
(ii) corriger la tension de Hall ($U_{Hall}$) de la tension de décalage ($U_{offset}$).

**15.** Procédé selon les revendications 12 à 14, **caractérisé par** les étapes consistant à :

(i) pour chaque capteur à effet Hall (42), détecter une sensibilité décrivant la dépendance de la tension de Hall par rapport au champ magnétique, et
(ii) corriger la position de l'influence de la sensibilité.

Fig. 1

Fig. 2a

Fig. 2b

Fig. 2

Fig. 3a

Fig. 3b

Fig. 3c

Fig. 3

Fig. 4a

Fig. 4b

Fig. 4

$$\Delta x' = \frac{d}{U'_{Hall,N-1} - U'_{Hall,N}} \cdot U'_{Hall,N}$$

$$x' = (N-1) \cdot d + \frac{d}{\Delta U'_{Hall}} \cdot U'_{Hall,N}$$

Fig. 5a

Fig. 5b

$$\Delta x'' = \frac{d}{U''_{Hall,N} - U''_{Hall,N+1}} \cdot U''_{Hall,N}$$

$$X'' = (N-1)\, d + \frac{d}{\Delta U''_{Hall}} \cdot U''_{Hall,N}$$

Fig.6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20140025182 A1
- US 20130245785 A1
- US 20160302946 A1
- US 20180116826 A1
- EP 2696814 B1
- US 7195645 B2
- US 20160238672 A
- GB 2367753 A